# EUROPEAN PATENT APPLICATION

(11) **EP 1 502 921 A1**
(43) Date of publication of application: **02.02.2005**
(21) Application number: 03016619.3
(22) Date of filing: 29.07.2003
(51) Int. Cl.: C07K 14/755, C12N 15/12, A61K 38/37, C12N 15/85

(54) **Recombinant mutated human factor VIII (FVIII) with improved stability**

(71) Applicant: ZLB Behring GmbH, 35041 Marburg (DE)
(72) Inventor: Hauser, Hans-Peter Dr., 35041 Marburg (DE); Weimer, Thomas Dr., 35075 Gladenbach (DE); Plantier, Jean-Luc, Dr., 69520 Grigny (FR); Ducasse, Cecille, 69001 Lyon (FR); Négrier, Claude, Prof. Dr., 69540 Irigny (FR)

(57) **Abstract**

The present invention relates to modified DNA sequences coding for biologically active recombinant human FVIII and its derivatives with improved stability, recombinant expression vectors containing such DNA sequences, host cells transformed with such recombinant expression vectors, and processes for the manufacture of the recombinant human FVIII and its derivatives. The invention also covers a transfer vector for use in human gene therapy which comprises such modified DNA sequences.

## Description

The present invention relates to modified DNA sequences coding for biologically active recombinant human FVIII and its derivatives with improved stability, recombinant expression vectors containing such DNA sequences, host cells transformed with such recombinant expression vectors, and processes for the manufacture of the recombinant human FVIII and its derivatives. The invention also covers a transfer vector for use in human gene therapy which comprises such modified DNA sequences.

Classic hemophilia or hemophilia A is the most common of the inherited bleeding disorders. It results from a chromosome X-linked deficiency of blood coagulation FVIII, and affects almost exclusively males with an incidence of between one and two individuals per 10.000. The X-chromosome defect is transmitted by female carriers who are not themselves hemophiliacs. The clinical manifestation of hemophilia A is an abnormal bleeding tendency and before treatment with FVIII concentrates was introduced the mean life span for a person with severe hemophilia was less than 20 years. The use of concentrates of FVIII from plasma has considerably improved the situation for the hemophilia patients. The mean life span has increased extensively, giving most of them the possibility to live a more or less normal life. However, there have been certain problems with the plasma derived concentrates and their use, the most serious of which have been the transmission of viruses. So far, viruses causing AIDS, hepatitis B, and non A non B hepatitis have hit the population seriously. Although different virus inactivation methods and new highly purified FVIII concentrates have recently been developed an inadvertent contamination can not be excluded. Also, the FVIII concentrates are fairly expensive because of the limited supply of human plasma raw material.

A FVIII product derived from recombinant material is likely to solve a large extent of the problems associated with the use of plasma derived FVIII concentrates for treatment for hemophilia A. However, the development of a recombinant FVIII has met some difficulties, for instance the problem of achieving production levels in sufficiently high yields, in particular regarding the full-length molecule.

In fresh plasma prepared in the presence of protease inhibitors, FVIII has been shown to have a molecular weight of 280 kDa and to be composed of two polypeptide chains of 200 kDa and 80 kDa, respectively (Andersson, L.-O., et al. (1986) Proc. Natl. Acad. Sci. USA 83, 2979-2983). These chains are held together by metal ion bridges. More or less proteolytically degraded forms of the FVIII molecule can be found as active fragments in FVIII material purified from commercial concentrates (Andersson, L.-O., et al. ibid.; Andersson, L.-O., et al. (1985) EP 0 197 901). The fragmented form of FVIII having molecular weights from 260 kDa down to 170 kDa, consists of one heavy chain with a molecular weight ranging from 180 kDa down to 90 kDa, where all variants have identical amino termini, in combination with one 80 kDa light chain. The amino-terminal region of the heavy chain is identical to that of the single chain FVIII polypeptide that can be deduced from the nucleotide sequence data of the FVIII cDNA (Wood, W.I., et al. (1984) Nature 312, 330-336; Vehar, G.A., et al. (1984) Nature 312, 337-342).

The smallest active form of FVIII with a molecular weight of 170 kDa, consisting of one 90 kDa and one 80 kDa chain, can be activated with thrombin to the same extent as the higher molecular weight forms, and thus represents a non activated form. It has also been shown to have full biological activity in vivo as tested in hemophilia dogs (Brinkhous, K.M., et al. (1985) Proc. Natl. Acad. Sci. USA 82, 8752-8756). Thus, the haemostatic effectiveness of the 170 kDa form is the same as for the high molecular weight forms of FVIII.

The fact that the middle heavily glycosylated region of the FVIII polypeptide chain residing between amino acids Arg-740 and Glu-1649 does not seem to be necessary for full biological activity has prompted several researchers to attempt to produce derivatives of recombinant FVIII lacking this region. This has been achieved by deleting a portion of the cDNA encoding the middle heavily glycosylated region of FVIII either entirely or partially.

For example, J.J. Toole, et al, reported the construction and expression of FVIII lacking amino acids 982 through 1562, and 760 through 1639 respectively (Proc. Natl. Acad. Sci. USA (1986) 83, 5939-5942). D.L. Eaton, et al. reported the construction and expression of FVIII lacking amino acids 797 through 1562 (Biochemistry (1986) 25, 8343-8347). R.J. Kaufman described the expression of FVIII lacking amino acids 741 through 1646 (PCT application No. WO 87/04187). N. Sarver, et al. reported the construction and expression of FVIII lacking amino acids 747 through 1560 (DNA (1987) 6, 553-564). M. Pasek reported the construction and expression of FVIII lacking amino acids 745 through 1562, and amino acids 741 through 1648, respectively (PCT application No. WO 88/00831). K.-D. Langner reported the construction and expression of FVIII lacking amino acids 816 through 1598, and amino acids 741 through 1689, respectively (Behring Inst. Mitt., (1988) No. 82, 16-25, EP 0 295 597). P. Meulien, et al., reported the construction and expression of FVIII lacking amino acids 868 through 1562, and amino acids 771 through 1666, respectively (Protein Engineering (1988) 2(4), 301-306, EP 0 303 540). When expressing these deleted forms of FVIII cDNA in mammalian cells the production level is typically 10 times higher as compared to full-length FVIII.

Recent results (Pipe S., Poster at the ISTH conference in Paris 2001, and oral presentation at the ISTH congress in Birmingham 2003) point to that the effect of increasing the FVIII expression yield by deleting the B-domain of FVIII is mainly due to enhanced mRNA levels, whereas the secretion efficacy is actually lower in a B-domain deleted FVIII molecule. It seems that retaining at least the N-terminal part of the B-domain with some of the N-glycosilation sites is beneficial for the secretion efficiency of the individual FVIII molecule once it entered the endoplasmatic reticulum.

FVIII is secreted into plasma as a heterodimer of a heavy chain (domains A1-A2-B). and a light chain (A3-C1-C2) associated through a non covalent divalent metal ion linkage between the A1- and A3 domains. In plasma, FVIII is stabilized by binding to von Willebrand factor.

Upon proteolytic activation by thrombin, FVIII is activated to a heterotrimer of 2 heavy chain fragments (A1, a 50 kDa fragment, and A2 a 43 kDa fragment) and the light chain (A3-C1-C2, a 73 kDa fragment). The active form of FVIII (FVIIIa) thus consists of an A1-subunit associated through the divalent metal ion linkage to a thrombin-cleaved A3-C1-C2 light chain and a free A2 subunit associated with the A1 domain. The dissociation of that free A2 subunit from the heterotrimer is thought to be the rate limiting step in FVIIIa inactivation after thrombin activation (Fay, P.J. et al, J. Biol. Chem. 266: 8957 (1991 ), Fay PJ & Smudzin TM, J. Biol. Chem. 267: 13246-50 (1992)). The half life of FVIIIa in plasma is only 2.1 minutes (Saenko et al., Vox Sang. 83: 89-96 (2002)). To enhance the half life of FVIIIa would result into a longer acting FVIIIa which would also translate into less frequent injections of such a FVIII preparation.
FVIII is administered i.v. to haemophilia patients who are on prophylactic treatment about 3 times per week due to the short plasma half life of FVIII of about 12 hours. It would thus be highly desirable to create a FVIII with increased plasma half life leading to a FVIII preparation which has to be administered less frequently. The present invention offers a solution to this by a modified FVIII molecule with an increased association of A2 to the A1/A3-C1-C2 which conveys to FVllla a longer functional half life.

The inactivation of FVIIIa through activated Protein C (APC) by cleavage at Arg336 and Arg562 is thought not to be the rate limiting step. Attempts have been made to create a FVIIIa which is inactivation resistant by covalently attaching the A2 domain to the A3 domain and by mutating the APC cleavage sites (Pipe and Kaufman, PNAS, 94:11851-11856).
Gale et. al. (Protein Science 2002, 11:2091-2101 ) published the stabilization of FV by covalently attaching the A3 domain to the A2 domain. They identified two neighbouring amino acids according to structural predictions, one on the A2 domain and the other being located on the A3 domain, and replaced these two amino acids with cysteine residues which formed a disulfide bridge during export into the endoplasmatic reticulum. The same approach was used as described in US 2003/0125232 and WO 02103024A2 to achieve a covalent attachment via disulfide bridges of the A3 and the A2 domain of FVIII. Such covalently attached FVIII mutants are absolutely stable for 40 minutes after activation whereas the activity of wild type FVIII quickly went down in an assay measuring APTT.
However it has been recently shown that constitutively high levels of FVIII might constitute a risk factor for thromboembolism (Kyrle 2003, Hamostasiologie 1:P.41-57). A FVIII with a covalent attachment of the A3 and the A2 domain therefore entails a potential for thrombotic events as it might be too resistant to inactivation so the life saving negative feedback mechanisms after hemostasis is achieved might be compromised.

It would thus be desirable to create a FVIII which displays a longer functional half life as compared to wild type FVIII but which is stabilized to a lesser extent as mutants with a covalent attachment between the A2 and the A3 domain.

It is therefore the purpose of this invention to create a FVIIIa in which the A2 domain is stabilized by non covalent means without completely blocking inactivation via dissociation of the A2 domain. This is very important, because this "limited stability" allows the almost impaired functioning of the coagulation cascade. "Limited stability" means that the plasma half-life of the modified biologically active recombinant human F VIII in its activated form is more than 3 minutes but not more than 30 minutes, preferably it is at least 10, but not more than 20 minutes.

Such stabilization could be achieved by a variety of approaches like the introduction of amino acids of the opposite charge, one in the A2 domain and the other in either the A1 or in the A3 domain which are a adjacent to each other. Other means of stabilizing the A2 domain would be the introduction of leucine zipper elements between the A2 and the A1 or the A3 domain or the creation of metal chelate sites leading to a metal ion stabilized A2 domain. These examples are given to illustrate the various approaches but are in no way limited to the mentioned examples. In general any modification which leads to an enhanced association of the A2 domain to A1 and/or the A3 domain excluding covalent attachment of the A2 domain to the A1 and/or the A3 domain would be part of the current invention.

According to US 2003/0125232 several pairs of amino acids capable of introducing a disulfide bond by replacing the respective amino acids with cysteines are disclosed. Two pairs, aa 662 (A2 domain) and aa1828 (A3 domain), and aa 664 and aa1826, respectively, appear close together and suited for replacement by charged amino acids of the invention, as the domains are assumed to present as antiparallel strands of amino acids and in addition, there are amino acids in the close vicinity which are already charged by nature and where the opposite amino acid requires replacement by an amino acid of opposite charge only.

aa656 Y T F K H K M V Y E D T aa667 (S 1/ Seq. ID 1)

aa1834 A K C D F E D K T P A M aa1823 (S 2/ Seq. ID 2)

A couple of mutants introducing amino acids of opposite charges were introduced in this region, in particular aa 657 to aa 666 and aa 1824 to 1833 as described in detail in the examples.

As a basis for introducing the mutations preferably a modified FVIII cDNA is used which comprises a first DNA segment coding for the amino acids 1 through 740 of the human FVIII and a second DNA segment coding for the amino acids 1649 through 2332 of the human FVIII. These two segments may be interconnected by a linker DNA segment, but the invention also encompasses introducing the mutations into full length FVIII.

Subject of the invention is therefore a modified human FVIII cDNA wherein mutations are inserted either in the wild-type FVIII cDNA or in a FVIII cDNA in which the B-domain is partially or completely deleted and may be replaced by a DNA linker segment, and the A2 domain is stabilized by mutations leading to a higher affinity of the A2 domain to the A1 and/or the A3 domain.

The production of FVIII proteins at high levels in suitable host cells, requires the assembly of the above-mentioned modified FVIII DNA's into efficient transcriptional units together with suitable regulatory elements in a recombinant expression vector, that can be propagated in E. coli according to methods known to those skilled in the art. Efficient transcriptional regulatory elements could be derived from viruses having animal cells as their natural hosts or from the chromosomal DNA of animal cells. Preferably, promoter-enhancer combinations derived from the Simian Virus 40, adenovirus, BK polyoma virus, human cytomegalovirus, or the long terminal repeat of Rous sarcoma virus, or promoter-enhancer combinations including strongly constitutively transcribed genes in animal cells like beta-actin or GRP78 can be used. In order to achieve stable high levels of mRNA transcribed from the FVIII DNA's, the transcriptional unit should contain in its 3'-proximal part a DNA region encoding a transcriptional termination-polyadenylation sequence. Preferably, this sequence is derived from the Simian Virus 40 early transcriptional region, the rabbit beta-globin gene, or the human tissue plasminogen activator gene.

The FVIII cDNA's are then integrated into the genome into a suitable host cell line for expression of the FVIII proteins. Preferably this cell line should be an animal cell-line of vertebrate origin in order to ensure correct folding, disulfide bond formation, asparagines-linked glycosylation and other post-translational modifications as well as secretion into the cultivation medium. Examples on other post-translational modifications are tyrosine O-sulfation, and proteolytic processing of the nascent polypeptide chain. Examples of cell lines that can be use are monkey COS-cells, mouse L-cells, mouse C127-cells, hamster BHK-21 cells, human embryonic kidney 293 cells, and preferentially CHO-cells.

The recombinant expression vector encoding the FVIII cDNA's can be introduced into an animal cell line in several different ways. For instance, recombinant expression vectors can be created from vectors based on different animal viruses, Examples of these are vectors based on baculovirus, vaccinia virus, adenovirus, and preferably bovine papilloma virus.

The transcription units encoding the FVIII DNA's can also be introduced into animal cells together with another recombinant gene which may function as a dominant selectable marker in these cells in order to facilitate the isolation of specific cell clones which have integrated the recombinant DNA into their genome. Examples of this type of dominant selectable marker genes are Tn5 aminoglycoside phosphotransferase, conferring resistance to Geneticin (G418), hygromycin phosphotransferase, conferring resistance to hygromycin, and puromycin acetyl transferase, conferring resistance to puromycin. The recombinant expression vector encoding such a selectable marker can reside either on the same vector as the one encoding the FVIII cDNA, or it can be encoded on a separate vector which is simultaneous!y introduced and integrated to the genome of the host cell, frequently resulting in a tight physical linkage between the different transcription units.

Other types of selectable marker genes which can be used together with the FVIII DNA's are based on various transcription units encoding dihydrofolate reductase (dhfr). After introduction of this type of gene into cells lacking endogenous dhfr-activity, preferentially CHO-cells (DUKX-B11, DG-44) it will enable these to grow in media lacking nucleosides. An example of such a medium is Ham's F12 without hypoxanthin, thymidin, and glycine. These dhfr-genes can be introduced together with the FVIII cDNA transcriptional units into CHO-cells of the above type, either linked on the same vector or on different vectors, thus creating dhfr-positive cell lines producing recombinant FVIII protein.

If the above cell lines are grown in the presence of the cytotoxic dhfr-inhibitor methotrexate, new cell lines resistant to methotrexate will emerge. These cell lines may produce recombinant FVIII protein at an increased rate due to the amplified number of linked dhfr and FVIII transcriptional units. When propagating these cell lines in increasing concentrations of methotrexate (1-10000 nM), new cell lines can be obtained which produce FVIII protein at very high rate.

The above cell lines producing FVIII protein can be grown on a large scale, either in suspension culture or on various solid supports. Examples of these supports are microcarriers based on dextran or collagen matrices, or solid supports in the form of hollow fibres or various ceramic materials. When grown in cell suspension culture or on microcarriers the culture of the above cell lines can be performed either as a bath culture or as a perfusion culture with continuous production of conditioned medium over extended periods of time. Thus, according to the present invention, the above cell lines are well suited for the development of an industrial process for the production of recombinant FVIII that can be isolated from human plasma.

The recombinant FVIII protein which accumulate in the medium of CHO-cells of the above type, can be concentrated and purified by a variety of biochemical and chromatographic methods, including methods utilizing differences in size, charge, hydrophobicity, solubility, specific affinity, etc. between the recombinant FVIII protein and other substances in the cell cultivation medium.

An example of such a purification is the adsorption of the recombinant FVIII protein to a monoclonal antibody which is immobilised on a solid support. After desorption, the FVIII protein can be further purified by a variety of chromatographic techniques based on the above properties.

The recombinant proteins with FVIII activity described in this invention can be formulated into pharmaceutical preparations for therapeutic use. The purified FVIII proteins may be dissolved in conventional physiologically compatible aqueous buffer solutions to which there may be added, optionally, pharmaceutical adjuvants to provide pharmaceutical preparations.

The modified FVIII DNA's of this invention may also be integrated into a transfer vector for use in the human gene therapy.

A further subject of this invention is a modified biologically active recombinant human FVIII with improved plasma half life of its activated form wherein mutations are inserted either in the wild-type FVIII or in a FVIII in which the B-domain is partially or completely deleted and replaced by a linker, and the A2 domain is stabilized by mutations leading to a higher affinity of the A2 domain to the A1 and/or the A3 domain.

The present invention will be further described more in detail in the following examples thereof. This description of specific embodiments of the invention will be made in conjunction with the appended figures.

### Generation of FVIII mutants

For the generation of FVIII mutants, a suitable subfragment of the FVIII cDNA (e.g. BamH1 - PfIMI, encompassing aminoacids 605 to 1889) was first subcloned into a suitable cloning vector to reduce subsequent sequencing efforts. Site directed mutagenesis was then performed with a commercially available mutagenesis kit (e.g. QuickChange SiteDirected Mutagenesis Kit (Stratagene) according to the manufacturer's instructions. Primers used for mutagenesis are listed in the attached sequence listing and below, where the mutagenic bases are indicated in bold letters.

The mutants generated are indicated in the following table.

| Clone | amino acid pairs and mutations* |
|---|---|
| 1 | M662K / D1828 |
| | Y664K / T1826D |
| | |
| 2 | M662K / D1828 |
| | Y664K / T1826D |
| | H660K / F1830D |
| | V663E / K1827 |
| | |
| 3 | H660K / F1830D |
| | V663E / K1827 |
| | |
| 4 | M662K / D1828 |
| | Y664K / T1826D |
| | H660K / F1830D |
| | V663E / K1827 |
| | T657D / K1833 |
| | D666 /A1824K |
| | |
| 5 | H660K / F1830D |
| | V663E / K1827 |
| | T657D / K1833 |
| | D666 / A1824K |

| | |
|---|---|
| *M662K indicates that the wild-type sequence at amino acid position was mutated from methionine to lysine; D1828 indicates that in this clone the wild-type sequence at amino acid position 1828 was not mutated, because it already presents a charged amino acid. | |

To generate clone 1 with the mutations indicated in the table above, the following mutations were introduced one after the other in the order given.
Mutation: M662K 
Forward primer (S 3/ Seq. ID 3):
5' GATATACCTTCAAACACAAAAAGGTCTATGAAGACACACTCAC3'
 
Reverse primer (S 4/ Seq. ID 4):
5´ GTGAGTGTGTCTTCATAGACCTTTTTGTGTTTGAAGGTATATC 3´
Mutation: Y664K 
Forward primer (S 5/ Seq. ID 5):
5' CCTTCAAACACAAAAAGGTCAAAGAAGACACACTCACCC TATTC 3'
 
Reverse primer (S 6/ Seq. ID 6):
5´ GAATAGGGTGAGTGTGTCTTCTTTGACCTTTTTGTGTTTGAAGG 3´
Mutation: T1826D

Forward primer (S 7/ Seq. ID 7)
5' GCAACATCATATGGCACCCGATAAAGATGAGTTTGACTGCAAAG 3'

Reverse primer (S 8/ Seq. ID 8)
5´ CTTTGCAGTCAAACTCATCTTTATCGGGTGCCATATGATGTTGC 3´

Based on clone 1 additional mutations one after the other in the order given were generated to construct clone 2:
Mutation: H660K 
Forward primer (S 9/ Seq. ID 9):
5' CTTCTTCTCTGGATATACCTTCAAAAGAAAAAGGTCAAAGAAGACACAC 3'
 
Reverse primer (S 10/ Seq. ID 10):
5´ GTGTGTCTTCTTTGACCTTTTTCTTTTTGAAGGTATATCCAGAGAAGAAG 3´
Mutation: V663K 
Forward primer (S 11/ Seq. ID 11):
5'GATATACCTTCAAAAAGAAAAAGAAGAAAGAAGACACACTCACCCTATTCCCT
ATTC 3'
 
Reverse primer (S 12/ Seq. ID 12):
5'GAATAGGGAATAGGGTGAGTGTGTCTTCTTTCTTCTTTTTCTTTTTGAAGGTAT
ATC 3'
Mutation: F1830D 
Forward primer (S 13/ Seq. ID 13):
5' GCACCCGATAAAGATGAGGATGACTGCAAAGCCTGGG 3'
 
Reverse primer (S 14/ Seq. ID 14):
5´ CCCAGGCTTTGCAGTCATCCTCATCTTTATCGGGTGC 3´
Mutation: K1827E

Forward primer (S 15/ Seq. ID 15):
5´ CATCATATGGCACCCGATGAAGATGAGGATGACTG 3´

Reverse primer (S 16/ Seq. ID 16):
5´ CAGTCATCCTCATCTTCATCGGGTGCCATATGATG 3´

To generate clone 3 with the mutations indicated in the table above, the following mutations were introduced one after the other in the order given.
Mutation: H660K 
Forward primer (S 17/ Seq. ID 17):
5' CTTCTCTGGATATACCTTCAAAAAGAAAATGGTCTATGAAGACACACTCAC 3'
 
Reverse primer (S 18/ Seq. ID 18):
5´ GTGAGTGTGTCTTCATAGACCATTTTCTTTTTGAAGGTATATCCAGAGAAG 3´
Mutation: V663K 
Forward primer (S 19/ Seq. ID 19):
5'CTCTGGATATACCTTCAAAAAGAAAATGAAGTATGAAGACACACTCACCCTAT
TCC 3'
 
Reverse primer (S 20/ Seq. ID 20):
5´GGAATAGGGTGAGTGTGTCTTCATACTTCATTTTCTTTTTGAAGGTATATCCA
GAG 3´
Mutation: F1830D 
Forward primer (S 21/ Seq. ID 21):
5´ GCACCCACTAAAGATGAGGATGACTGCAAAGCCTGG 3´
 
Reverse primer (S 22/ Seq. ID 22):
5´ CCAGGCTTTGCAGTCATCCTCATCTTTAGTGGGTGC 3´
Mutation: K1827E

Forward primer (S 23/ Seq. ID 23):
5´ CATCATATGGCACCCACTGAAGATGAGGATGACTG 3´

Reverse primer (S 24/ Seq. ID 24):
5´ CAGTCATCCTCATCTTCAGTGGGTGCCATATGATG 3´

Based on clone 2 additional mutations one after the other in the order given were generated to construct clone 4:
Mutation: T657D 
Forward primer (S 25/ Seq. ID 25):
5'CTGTCTTCTTCTCTGGATATGACTTCAAAAAGAAAAAGAAGAAAGAAGACACA
C 3'
 
Reverse primer (S 26/ Seq. ID 26):
5'GTGTGTCTTCTTTCTTCTTTTTCTTTTTGAAGTCATATCCAGAGAAGAAGACAG
3'
Mutation: A1824K

Mutation: [?]

`Forward primer (S 27/ Seq. ID 27):

5´ GGAAAGTGCAACATCATATGAAACCCGATGAAGATGAGGATG 3´

Reverse primer (S 28/ Seq. ID 28):
5´ CATCCTCATCTTCATCGGGTTTCATATGATGTTGCACTTTCC 3´

Based on clone 3 additional mutations one after the other in the order given were generated to construct clone 5:
Mutation: T657D 
[?]
Forward primer (S 29/ Seq. ID 29):
5'CTGTCTTCTTCTCTGGATATGACTTCAAAAAGAAAATGAAGTATGAAGACAC3'
 
Reverse primer (S 30/ Seq. ID 30):
5´GTGTCTTCATACTTCATTTTCTTTTTGAAGTCATATCCAGAGAAGAAGACAG3´
Mutation: A1824K

Forward primer (S 31/ Seq. ID 31):
5´GGAAAGTGCAACATCATATGAAACCCACTGAAGATGAGGATG3´

Reverse primer (S 32/ Seq. ID 32):
5´CATCCTCATCTTCAGTGGGTTTCATATGATGTTGCACTTTCC 3´

After clone isolation and sequence verification mutant subfragments were reinserted into the respective expression vector.

### Examples

### 1) Expression of FVIII mutants

Transfection of FVIII mutant clones and expression of the mutant FVIII molecules is done as described previously and known to those skilled in the art (e.g. Plantier JL et al. Thromb. Haemost. 86:596-603 (2001)).

### 2) Measuring affinity of A2 subunit for A1/A3-C1-C2

The increased affinity of the A2 subunit for the A1/A3-C1-C2 can be measured as previously described by functional assays (Fay PJ & Smudzin TM. J. Biol. Chem. 267:13246-50 (1992); Lollar P et al. J. Biol. Chem. 267:23652-57 (1992)) as well as a physical assay employing surface plasmon resonance (Persson E et al. Biochemistry 34:12775-81 (1995)).

### 3) Measuring the functional half life of FVIIIa

The increased functional half life can be determined in vitro as shown in figure 5 of US 2003/0125232 or as published by Sandberg (Thromb. Haemost. 2001 ;85(1 ):93-100)

The sequence of the human FVIII is shown in sequence 31.

SEQUENCE LISTING

<110>  Aventis Behring GmbH

<120>  Modified cDNA Factor VIII and its Derivatives

<130>  2003/M012(A078)

<140>  w/o
<141>  2003-07-30

<160>  33

<170>  PatentIn version 3.1

<210>  1
<211>  12
<212>  PRT
<213>  Artificial Sequence

<220>
<223>  human
<220>
<221>  PEPTIDE
<222>  (1)..(12)
<223>

<400>  1

Tyr Thr Phe Lys His Lys Met Val Tyr Glu Asp Thr
1               5                   10

<210>  2
<211>  12
<212>  PRT
<213>  Artificial Sequence

<220>

<223> human

<220>

<221> PEPTIDE

<222> (1)..(12)

<223>

<400> 2

Ala Lys Cys Asp Phe Glu Asp Lys Thr Pro Ala Met
1               5                   10

<210> 3

<211> 43

<212> DNA

<213> Artificial Sequence

<220>

<223> human

<400> 3
gatatacctt caaacacaaa aaggtctatg aagacacact cac                43

<210> 4

<211> 43

<212> DNA

<213> Artificial Sequence

<220>

<223> human

<220>

<221> primer reverse

<222> (1)..(43)

<223>

<400> 4
gtgagtgtgt cttcatagac ctttttgtgt ttgaaggtat atc                43

<210> 5

<211> 44

<212> DNA

<213> Artificial Sequence

<220>

<223> human

<220>

<221> primerforward

<222> (1)..(43)

<223>

<400> 5
ccttcaaaca caaaaaggtc aaagaagaca cactcaccct attc 44

<210> 6

<211> 44

<212> DNA

<213> Artificial Sequence

<220>

<223> human

<220>

<221> primerreverse

<222> (1)..(43)

<223>

<220>

<221> primerreverse

<222> (1)..(44)

<223>

<400> 6
gaatagggtg agtgtgtctt ctttgacctt tttgtgtttg aagg 44

<210> 7

<211>  44

<212>  DNA

<213>  Artificial Sequence

<220>

<223>  human

<220>

<221>  primerforward

<222>  (1)..(43)

<223>

<220>

<221>  primerforward

<222>  (1)..(44)

<223>

<400>  7
gcaacatcat atggcacccg ataaagatga gtttgactgc aaag                    44

<210>  8

<211>  44

<212>  DNA

<213>  Artificial Sequence

<220>

<223>  human

<220>

<221>  primerreverse

<222>  (1)..(44)

<223>

<400>  8
ctttgcagtc aaactcatct ttatcgggtg ccatatgatg ttgc                    44

<210>  9

<211>  50

<212>  DNA
<213>  Artificial Sequence

<220>
<223>  human
<220>
<221>  primerforward
<222>  (1)..(50)
<223>

<400>  9
cttcttctct ggatatacct tcaaaaagaa aaaggtcaaa gaagacacac                50

<210>  10
<211>  50
<212>  DNA
<213>  Artificial Sequence

<220>
<223>  human
<220>
<221>  primerreverse
<222>  (1)..(50)
<223>

<400>  10
gtgtgtcttc tttgaccttt ttctttttga aggtatatcc agagaagaag                50

<210>  11
<211>  57
<212>  DNA
<213>  Artificial Sequence

<220>
<223>  human
<220>

<221>  forwardprimer
<222>  (1)..(57)
<223>

<400>  11
gatatacctt caaaaagaaa aagaagaaag aagacacact caccctattc cctattc          57

<210>  12
<211>  57
<212>  DNA
<213>  Artificial Sequence

<220>
<223>  human
<220>
<221>  primerreverse
<222>  (1)..(57)
<223>

<400>  12
gaatagggaa tagggtgagt gtgtcttctt tcttcttttt ctttttgaag gtatatc          57

<210>  13
<211>  37
<212>  DNA
<213>  Artificial Sequence

<220>
<223>  human
<220>
<221>  primerforward
<222>  (1)..(37)
<223>

<400>  13
gcacccgata aagatgagga tgactgcaaa gcctggg                                37

<210>  14

<211>  37

<212>  DNA

<213>  Artificial Sequence

<220>

<223>  human

<220>

<221>  primerreverse

<222>  (1)..(37)

<223>

<400>  14
cccaggcttt gcagtcatcc tcatctttat cgggtgc                                37

<210>  15

<211>  35

<212>  DNA

<213>  Artificial Sequence

<220>

<223>  human

<220>

<221>  primerforward

<222>  (1)..(35)

<223>

<400>  15
catcatatgg cacccgatga agatgaggat gactg                                  35

<210>  16

<211>  35

<212>  DNA

<213>  Artificial Sequence

<220>

<223>  human

<220>

<221>  Reverse primer

<222>  (1)..(35)

<223>

<400>  16
cagtcatcct catcttcatc gggtgccata tgatg                                35

<210>  17

<211>  51

<212>  DNA

<213>  Artificial Sequence

<220>

<223>  human

<220>

<221>  Forward primer

<222>  (1)..(35)

<223>

<220>

<221>  Forward primer

<222>  (1)..(51)

<223>

<400>  17
cttctctgga tataccttca aaaagaaaat ggtctatgaa gacacactca c              51

<210>  18

<211>  51

<212>  DNA

<213>  Artificial Sequence

<220>

<223>  human

<220>

<221>  Reverse primer

<222>  (1)..(51)

<223>

<400>  18
gtgagtgtgt cttcatagac cattttcttt ttgaaggtat atccagagaa g          51

<210>  19

<211>  56

<212>  DNA

<213>  Artificial Sequence

<220>

<223>  human

<220>

<221>  Forward primer

<222>  (1)..(51)

<223>

<400>  19
ctctggatat accttcaaaa agaaaatgaa gtatgaagac acactcaccc tattcc          56

<210>  20

<211>  56

<212>  DNA

<213>  Artificial Sequence

<220>

<223>  human

<220>

<221>  Reverse primer

<222>  (1)..(51)

<223>

<400>  20
ggaatagggt gagtgtgtct tcatacttca ttttcttttt gaaggtatat ccagag          56

<210> 21

<211> 36

<212> DNA

<213> Artificial Sequence

<220>

<223> human

<220>

<221> Forward primer

<222> (1)..(35)

<223>

<220>

<221> Forward primer

<222> (1)..(36)

<223>

<400> 21
gcacccacta aagatgagga tgactgcaaa gcctgg 36

<210> 22

<211> 36

<212> DNA

<213> Artificial Sequence

<220>

<223> human

<220>

<221> Reverse primer

<222> (1)..(36)

<223>

<400> 22
ccaggctttg cagtcatcct catctttagt gggtgc 36

<210> 23

<211> 35

<212> DNA

<213> Artificial Sequence

<220>

<223> human

<400> 23
catcatatgg cacccactga agatgaggat gactg 35

<210> 24

<211> 35

<212> DNA

<213> Artificial Sequence

<220>

<223> human

<220>

<221> Reverse primer

<222> (1)..(35)

<223>

<400> 24
cagtcatcct catcttcagt gggtgccata tgatg 35

<210> 25

<211> 54

<212> DNA

<213> Artificial Sequence

<220>

<223> human

<220>

<221> Forward primer

<222> (1)..(54)

<223>

<400>  25
ctgtcttctt ctctggatat gacttcaaaa agaaaaagaa gaaagaagac acac                54

<210>  26

<211>  54

<212>  DNA

<213>  Artificial Sequence


<220>

<223>  human

<220>

<221>  Reverse primer

<222>  (1)..(54)

<223>


<400>  26
gtgtgtcttc tttcttcttt ttctttttga agtcatatcc agagaagaag acag                54

<210>  27

<211>  42

<212>  DNA

<213>  Artificial Sequence


<220>

<223>  human

<220>

<221>  Forward primer

<222>  (1)..(42)

<223>


<400>  27
ggaaagtgca acatcatatg aaacccgatg aagatgagga tg                42

<210>  28

<211>  42

<212>  DNA

<213>  Artificial Sequence

<220>
<223>  human
<220>
<221>  Reverse primer
<222>  (1)..(42)
<223>

<400>  28
catcctcatc ttcatcgggt ttcatatgat gttgcacttt cc                    42

<210>  29
<211>  52
<212>  DNA
<213>  Artificial Sequence

<220>
<223>  human
<220>
<221>  Forward primer
<222>  (1)..(52)
<223>

<400>  29
ctgtcttctt ctctggatat gacttcaaaa agaaaatgaa gtatgaagac ac          52

<210>  30
<211>  52
<212>  DNA
<213>  Artificial Sequence

<220>
<223>  human
<220>
<221>  Reverse primer

<222>  (1)..(52)

<223>

<400>  30
gtgtcttcat acttcatttt ctttttgaag tcatatccag agaagaagac ag          52

<210>  31

<211>  42

<212>  DNA

<213>  Artificial Sequence

<220>

<223>  human

<220>

<221>  Forward primer

<222>  (1)..(42)

<223>

<400>  31
ggaaagtgca acatcatatg aaacccactg aagatgagga tg          42

<210>  32

<211>  42

<212>  DNA

<213>  Artificial Sequence

<220>

<223>  human

<220>

<221>  Reverse primer

<222>  (1)..(42)

<223>

<400>  32
catcctcatc ttcagtgggt ttcatatgat gttgcacttt cc          42

<210>  33

<211>  2332

<212>  PRT

<213>  human

<220>

<221>  CHAIN

<222>  (1)..(2332)

<223>

<400>  33

Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Arg Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Leu Gly Pro Pro Ser Met Pro Val His
            900                 905                 910

Tyr Asp Ser Gln Leu Asp Thr Thr Leu Phe Gly Lys Lys Ser Ser Pro
        915                 920                 925

Leu Thr Glu Ser Gly Gly Pro Leu Ser Leu Ser Glu Glu Asn Asn Asp
    930                 935                 940

Ser Lys Leu Leu Glu Ser Gly Leu Met Asn Ser Gln Glu Ser Ser Trp
945                 950                 955                 960

Gly Lys Asn Val Ser Ser Thr Glu Ser Gly Arg Leu Phe Lys Gly Lys
                965                 970                 975

Arg Ala His Gly Pro Ala Leu Leu Thr Lys Asp Asn Ala Leu Phe Lys
            980                 985                 990

Val Ser Ile Ser Leu Leu Lys Thr Asn Lys Thr Ser Asn Asn Ser Ala
        995                 1000                1005

Thr Asn Arg Lys Thr His Ile Asp Gly Pro Ser Leu Leu Ile Glu
    1010                1015                1020

Asn Ser Pro Ser Val Trp Gln Asn Ile Leu Glu Ser Asp Thr Glu
    1025                1030                1035

Phe Lys Lys Val Thr Pro Leu Ile His Asp Arg Met Leu Met Asp
    1040                1045                1050

Lys Asn Ala Thr Ala Leu Arg Leu Asn His Met Ser Asn Lys Thr
    1055                1060                1065

Thr Ser Ser Lys Asn Met Glu Met Val Gln Gln Lys Lys Glu Gly
    1070                1075                1080

Pro Ile Pro Pro Asp Ala Gln Asn Pro Asp Met Ser Phe Phe Lys
    1085                1090                1095

Met Leu Phe Leu Pro Glu Ser Ala Arg Trp Ile Gln Arg Thr His
    1100                1105                1110

Gly Lys Asn Ser Leu Asn Ser Gly Gln Gly Pro Ser Pro Lys Gln
    1115                1120                1125

Leu Val Ser Leu Gly Pro Glu Lys Ser Val Glu Gly Gln Asn Phe
    1130                1135                1140

Leu Ser Glu Lys Asn Lys Val Val Val Gly Lys Gly Glu Phe Thr
    1145                1150                1155

Lys Asp Val Gly Leu Lys Glu Met Val Phe Pro Ser Ser Arg Asn
    1160                1165                1170

Leu Phe Leu Thr Asn Leu Asp Asn Leu His Glu Asn Asn Thr His
    1175                1180                1185

Asn Gln Glu Lys Lys Ile Gln Glu Glu Ile Glu Lys Lys Glu Thr
    1190                1195                1200

Leu Ile Gln Glu Asn Val Val Leu Pro Gln Ile His Thr Val Thr
    1205                1210                1215

Gly Thr Lys Asn Phe Met Lys Asn Leu Phe Leu Leu Ser Thr Arg
    1220                1225                1230

Gln Asn Val Glu Gly Ser Tyr Asp Gly Ala Tyr Ala Pro Val Leu
    1235                1240                1245

Gln Asp Phe Arg Ser Leu Asn Asp Ser Thr Asn Arg Thr Lys Lys
    1250                1255                1260

His Thr Ala His Phe Ser Lys Lys Gly Glu Glu Glu Asn Leu Glu
    1265                    1270                    1275

Gly Leu Gly Asn Gln Thr Lys Gln Ile Val Glu Lys Tyr Ala Cys
    1280                    1285                    1290

Thr Thr Arg Ile Ser Pro Asn Thr Ser Gln Gln Asn Phe Val Thr
    1295                    1300                    1305

Gln Arg Ser Lys Arg Ala Leu Lys Gln Phe Arg Leu Pro Leu Glu
    1310                    1315                    1320

Glu Thr Glu Leu Glu Lys Arg Ile Ile Val Asp Asp Thr Ser Thr
    1325                    1330                    1335

Gln Trp Ser Lys Asn Met Lys His Leu Thr Pro Ser Thr Leu Thr
    1340                    1345                    1350

Gln Ile Asp Tyr Asn Glu Lys Glu Lys Gly Ala Ile Thr Gln Ser
    1355                    1360                    1365

Pro Leu Ser Asp Cys Leu Thr Arg Ser His Ser Ile Pro Gln Ala
    1370                    1375                    1380

Asn Arg Ser Pro Leu Pro Ile Ala Lys Val Ser Ser Phe Pro Ser
    1385                    1390                    1395

Ile Arg Pro Ile Tyr Leu Thr Arg Val Leu Phe Gln Asp Asn Ser
    1400                    1405                    1410

Ser His Leu Pro Ala Ala Ser Tyr Arg Lys Lys Asp Ser Gly Val
    1415                    1420                    1425

Gln Glu Ser Ser His Phe Leu Gln Gly Ala Lys Lys Asn Asn Leu
    1430                    1435                    1440

Ser Leu Ala Ile Leu Thr Leu Glu Met Thr Gly Asp Gln Arg Glu
    1445                    1450                    1455

Val Gly Ser Leu Gly Thr Ser Ala Thr Asn Ser Val Thr Tyr Lys
    1460                    1465                    1470

Lys Val Glu Asn Thr Val Leu Pro Lys Pro Asp Leu Pro Lys Thr
    1475                    1480                    1485

Ser Gly Lys Val Glu Leu Leu Pro Lys Val His Ile Tyr Gln Lys
    1490                    1495                    1500

Asp Leu Phe Pro Thr Glu Thr Ser Asn Gly Ser Pro Gly His Leu
    1505                    1510                    1515

Asp Leu Val Glu Gly Ser Leu Leu Gln Gly Thr Glu Gly Ala Ile
    1520                1525                1530

Lys Trp Asn Glu Ala Asn Arg Pro Gly Lys Val Pro Phe Leu Arg
    1535                1540                1545

Val Ala Thr Glu Ser Ser Ala Lys Thr Pro Ser Lys Leu Leu Asp
    1550                1555                1560

Pro Leu Ala Trp Asp Asn His Tyr Gly Thr Gln Ile Pro Lys Glu
    1565                1570                1575

Glu Trp Lys Ser Gln Glu Lys Ser Pro Glu Lys Thr Ala Phe Lys
    1580                1585                1590

Lys Lys Asp Thr Ile Leu Ser Leu Asn Ala Cys Glu Ser Asn His
    1595                1600                1605

Ala Ile Ala Ala Ile Asn Glu Gly Gln Asn Lys Pro Glu Ile Glu
    1610                1615                1620

Val Thr Trp Ala Lys Gln Gly Arg Thr Glu Arg Leu Cys Ser Gln
    1625                1630                1635

Asn Pro Pro Val Leu Lys Arg His Gln Arg Glu Ile Thr Arg Thr
    1640                1645                1650

Thr Leu Gln Ser Asp Gln Glu Glu Ile Asp Tyr Asp Asp Thr Ile
    1655                1660                1665

Ser Val Glu Met Lys Lys Glu Asp Phe Asp Ile Tyr Asp Glu Asp
    1670                1675                1680

Glu Asn Gln Ser Pro Arg Ser Phe Gln Lys Lys Thr Arg His Tyr
    1685                1690                1695

Phe Ile Ala Ala Val Glu Arg Leu Trp Asp Tyr Gly Met Ser Ser
    1700                1705                1710

Ser Pro His Val Leu Arg Asn Arg Ala Gln Ser Gly Ser Val Pro
    1715                1720                1725

Gln Phe Lys Lys Val Val Phe Gln Glu Phe Thr Asp Gly Ser Phe
    1730                1735                1740

Thr Gln Pro Leu Tyr Arg Gly Glu Leu Asn Glu His Leu Gly Leu
    1745                1750                1755

Leu Gly Pro Tyr Ile Arg Ala Glu Val Glu Asp Asn Ile Met Val
    1760                1765                1770

Thr Phe Arg Asn Gln Ala Ser Arg Pro Tyr Ser Phe Tyr Ser Ser
    1775                1780                1785

Leu Ile Ser Tyr Glu Glu Asp Gln Arg Gln Gly Ala Glu Pro Arg
    1790                1795                1800

Lys Asn Phe Val Lys Pro Asn Glu Thr Lys Thr Tyr Phe Trp Lys
    1805                1810                1815

Val Gln His His Met Ala Pro Thr Lys Asp Glu Phe Asp Cys Lys
    1820                1825                1830

Ala Trp Ala Tyr Phe Ser Asp Val Asp Leu Glu Lys Asp Val His
    1835                1840                1845

Ser Gly Leu Ile Gly Pro Leu Leu Val Cys His Thr Asn Thr Leu
    1850                1855                1860

Asn Pro Ala His Gly Arg Gln Val Thr Val Gln Glu Phe Ala Leu
    1865                1870                1875

Phe Phe Thr Ile Phe Asp Glu Thr Lys Ser Trp Tyr Phe Thr Glu
    1880                1885                1890

Asn Met Glu Arg Asn Cys Arg Ala Pro Cys Asn Ile Gln Met Glu
    1895                1900                1905

Asp Pro Thr Phe Lys Glu Asn Tyr Arg Phe His Ala Ile Asn Gly
    1910                1915                1920

Tyr Ile Met Asp Thr Leu Pro Gly Leu Val Met Ala Gln Asp Gln
    1925                1930                1935

Arg Ile Arg Trp Tyr Leu Leu Ser Met Gly Ser Asn Glu Asn Ile
    1940                1945                1950

His Ser Ile His Phe Ser Gly His Val Phe Thr Val Arg Lys Lys
    1955                1960                1965

Glu Glu Tyr Lys Met Ala Leu Tyr Asn Leu Tyr Pro Gly Val Phe
    1970                1975                1980

Glu Thr Val Glu Met Leu Pro Ser Lys Ala Gly Ile Trp Arg Val
    1985                1990                1995

Glu Cys Leu Ile Gly Glu His Leu His Ala Gly Met Ser Thr Leu
    2000                2005                2010

Phe Leu Val Tyr Ser Asn Lys Cys Gln Thr Pro Leu Gly Met Ala
    2015                2020                2025

Ser Gly His Ile Arg Asp Phe Gln Ile Thr Ala Ser Gly Gln Tyr
    2030                2035                2040

Gly Gln Trp Ala Pro Lys Leu Ala Arg Leu His Tyr Ser Gly Ser
    2045                2050                2055

Ile Asn Ala Trp Ser Thr Lys Glu Pro Phe Ser Trp Ile Lys Val
    2060                2065                2070

Asp Leu Leu Ala Pro Met Ile Ile His Gly Ile Lys Thr Gln Gly
    2075                2080                2085

Ala Arg Gln Lys Phe Ser Ser Leu Tyr Ile Ser Gln Phe Ile Ile
    2090                2095                2100

Met Tyr Ser Leu Asp Gly Lys Lys Trp Gln Thr Tyr Arg Gly Asn
    2105                2110                2115

Ser Thr Gly Thr Leu Met Val Phe Phe Gly Asn Val Asp Ser Ser
    2120                2125                2130

Gly Ile Lys His Asn Ile Phe Asn Pro Pro Ile Ile Ala Arg Tyr
    2135                2140                2145

Ile Arg Leu His Pro Thr His Tyr Ser Ile Arg Ser Thr Leu Arg
    2150                2155                2160

Met Glu Leu Met Gly Cys Asp Leu Asn Ser Cys Ser Met Pro Leu
    2165                2170                2175

Gly Met Glu Ser Lys Ala Ile Ser Asp Ala Gln Ile Thr Ala Ser
    2180                2185                2190

Ser Tyr Phe Thr Asn Met Phe Ala Thr Trp Ser Pro Ser Lys Ala
    2195                2200                2205

Arg Leu His Leu Gln Gly Arg Ser Asn Ala Trp Arg Pro Gln Val
    2210                2215                2220

Asn Asn Pro Lys Glu Trp Leu Gln Val Asp Phe Gln Lys Thr Met
    2225                2230                2235

Lys Val Thr Gly Val Thr Thr Gln Gly Val Lys Ser Leu Leu Thr
    2240                2245                2250

Ser Met Tyr Val Lys Glu Phe Leu Ile Ser Ser Ser Gln Asp Gly
    2255                2260                2265

His Gln Trp Thr Leu Phe Phe Gln Asn Gly Lys Val Lys Val Phe
    2270                2275                2280

Gln Gly Asn Gln Asp Ser Phe Thr Pro Val Val Asn Ser Leu Asp
    2285                2290                2295

Pro Pro Leu Leu Thr Arg Tyr Leu Arg Ile His Pro Gln Ser Trp
    2300                2305                2310

Val His Gln Ile Ala Leu Arg Met Glu Val Leu Gly Cys Glu Ala
    2315                2320                2325

Gln Asp Leu Tyr
    2330

S 33 (Seq ID 33):

ATRRYYLGAV ELSWDYMQSD LGELPVDARF PPRVPKSFPF NTSVVYKKTL FVEFTDHLFN 60

IAKPRPPWMG LLGPTIQAEV YDTVVITLKN MASHPVSLHA VGVSYWKASE GAEYDDQTSQ 120

REKEDDKVFP GGSHTYVWQV LKENGPMASD PLCLTYSYLS HVDLVKDLNS GLIGALLVCR 180

EGSLAKEKTQ TLHKFILLFA VFDEGKSWHS ETKNSLMQDR DAASARAWPK MHTVNGYVNR 240

SLPGLIGCHR KSVYWHVIGM GTTPEVHSIF LEGHTFLVRN HRQASLEISP ITFLTAQTLL 300

MDLGQFLLFC HISSHQHDGM EAYVKVDSCP EEPQLRMKNN EEAEDYDDDL TDSEMDVVRF 360

DDDNSPSFIQ IRSVAKKHPK TWVHYIAAEE EDWDYAPLVL APDDRSYKSQ YLNNGPQRIG 420

RKYKKVRFMA YTDETFKTRE AIQHESGILG PLLYGEVGDT LLIIFKNQAS RPYNIYPHGI 480

TDVRPLYSRR LPKGVKHLKD FPILPGEIFK YKWTVTVEDG PTKSDPRCLT RYYSSFVNME 540

RDLASGLIGP LLICYKESVD QRGNQIMSDK RNVILFSVFD ENRSWYLTEN IQRFLPNPAG 600

VQLEDPEFQA SNIMHSINGY VFDSLQLSVC LHEVAYWYIL SIGAQTDFLS VFFSGYTFKH 660

KMVYEDTLTL FPFSGETVFM SMENPGLWIL GCHNSDFRNR GMTALLKVSS CDKNTGDYYE 720

DSYEDISAYL LSKNNAIEPR SFSQNSRHRS TRQKQFNATT IPENDIEKTD PWFAHRTPMP 780

KIQNVSSSDL LMLLRQSPTP HGLSLSDLQE AKYETFSDDP SPGAIDSNNS LSEMTHFRPQ 840

LHHSGDMVFT PESGLQLRLN EKLGTTAATE LKKLDFKVSS TSNNLISTIP SDNLAAGTDN 900

TSSLGPPSMP VHYDSQLDTT LFGKKSSPLT ESGGPLSLSE ENNDSKLLES GLMNSQESSW 960

GKNVSSTESG RLFKGKRAHG PALLTKDNAL FKVSISLLKT NKTSNNSATN RKTHIDGPSL 1020

LIENSPSVWQ NILESDTEFK KVTPLIHDRM LMDKNATALR LNHMSNKTTS SKNMEMVQQK 1080

KEGPIPPDAQ NPDMSFFKML FLPESARWIQ RTHGKNSLNS GQGPSPKQLV SLGPEKSVEG 1140

QNFLSEKNKV VVGKGEFTKD VGLKEMVFPS SRNLFLTNLD NLHENNTHNQ EKKIQEEIEK 1200

KETLIQENVV LPQIHTVTGT KNFMKNLFLL STRQNVEGSY DGAYAPVLQD FRSLNDSTNR 1260

TKKHTAHFSK KGEEENLEGL GNQTKQIVEK YACTTRISPN TSQQNFVTQR SKRALKQFRL 1320

PLEETELEKR IIVDDTSTQW SKNMKHLTPS TLTQIDYNEK EKGAITQSPL SDCLTRSHSI 1380

PQANRSPLPI AKVSSFPSIR PIYLTRVLFQ DNSSHLPAAS YRKKDSGVQE SSHFLQGAKK 1440

NNLSLAILTL EMTGDQREVG SLGTSATNSV TYKKVENTVL PKPDLPKTSG KVELLPKVHI 1500

YQKDLFPTET SNGSPGHLDL VEGSLLQGTE GAIKWNEANR PGKVPFLRVA TESSAKTPSK 1560

LLDPLAWDNH YGTQIPKEEW KSQEKSPEKT AFKKKDTILS LNACESNHAI AAINEGQNKP 1620

EIEVTWAKQG RTERLCSQNP PVLKRHQREI TRTTLQSDQE EIDYDDTISV EMKKEDFDIY 1680

DEDENQSPRS FQKKTRHYFI AAVERLWDYG MSSSPHVLRN RAQSGSVPQF KKVVFQEFTD 1740

GSFTQPLYRG ELNEHLGLLG PYIRAEVEDN IMVTFRNQAS RPYSFYSSLI SYEEDQRQGA 1800

EPRKNFVKPN ETKTYFWKVQ HHMAPTKDEF DCKAWAYFSD VDLEKDVHSG LIGPLLVCHT 1860

NTLNPAHGRQ VTVQEFALFF TIFDETKSWY FTENMERNCR APCNIQMEDP TFKENYRFHA 1920

INGYIMDTLP GLVMAQDQRI RWYLLSMGSN ENIHSIHFSG HVFTVRKKEE YKMALYNLYP 1980

GVFETVEMLP SKAGIWRVEC LIGEHLHAGM STLFLVYSNK CQTPLGMASG HIRDFQITAS 2040

GQYGQWAPKL ARLHYSGSIN AWSTKEPFSW IKVDLLAPMI IHGIKTQGAR QKFSSLYISQ 2100

FIIMYSLDGK KWQTYRGNST GTLMVFFGNV DSSGIKHNIF NPPIIARYIR LHPTHYSIRS 2160

TLRMELMGCD LNSCSMPLGM ESKAISDAQI TASSYFTNMF ATWSPSKARL HLQGRSNAWR 2220

PQVNNPKEWL QVDFQKTMKV TGVTTQGVKS LLTSMYVKEF LISSSQDGHQ WTLFFQNGKV 2280

KVFQGNQDSF TPVVNSLDPP LLTRYLRIHP QSWVHQIALR MEVLGCEAQD LY 2332

## Claims

1. Modified biologically active recombinant human FVIII with improved stability of its activated form wherein mutations are inserted, **characterised in that** they enhance the affinity of the A2 domain to the A1 domain and/or the A3 domain.

2. Modified biologically active recombinant human FVIII according to claim 1 wherein such mutations provide additional ionic interdomain interactions.

3. Modified biologically active recombinant human FVIII according to claim 1 or 2, wherein the plasma half life of its activated form is more than 3 minutes, preferably more than 10 minutes and less than 30 minutes.

4. Modified biologically active recombinant human FVIII according to claim 1 or 2, wherein mutations are inserted either in the wild-type FVIII or in a FVIII in which the B-domain is partially or completely deleted and may be replaced by a linker.

5. Modified human FVIII cDNA which can be used to express a human FVIII according to claim 1 to 4 **characterised in that** mutations are inserted into one or several codons of the human FVili cDNA.

6. Modified human FVIII cDNA according to claim 5, wherein mutations are inserted either in the wild-type FVIII cDNA or in a FVIII cDNA in which the B-domain is partially or completely deleted and may be replaced by a DNA linker segment.

7. Process for the recombinant production of a modified human FVIII as claimed in claim 1 or 2 either in cell suspension or on a solid support as a bath cell culture or as a perfusion cell culture with continuous production of a conditioned medium **characterised in that** the FVIII proteins, which are expressed by a suitable host cell line are purified by chromatographic methods.

8. Process as claimed in claim 7, **characterised in that** the transcription units encoding the modified FVIII cDNA of claims 5 or 6 contain a dominant selectable marker in order to facilitate the isolation of specific cell clones which have integrated said specific c-DNA into their genome.

9. Host cell line for expression of the FVIII proteins of claim 1 or 2, **characterised in that** it is an animal cell line of vertebrate origin which contains the FVIII cDNA of claim 5 integrated into its genome.

10. Pharmaceutical composition, **characterised in that** it comprises a modified biologically active recombinant human FVIII of claim 1 or 2.

11. Vector for gene therapy of hemophilia A, **characterized in that** it contains a modified FVIII cDNA as claimed in claim 5.
